# EUROPEAN PATENT SPECIFICATION

(11) **EP 3 442 628 B1**
(45) Date of publication and mention of the grant of the patent: **20.03.2024**
(21) Application number: 16898835.0
(22) Date of filing: 15.04.2016
(51) Int. Cl.: B05B 7/04, A61M 11/00, A61M 11/06, A61M 15/00, B05B 7/00, B05B 7/16, B05B 15/50

(54) **AEROSOLIZING NOZZLE AND METHOD OF OPERATING SUCH AEROSOLIZING NOZZLE**
AEROSOLBILDENDE DÜSE UND VERFAHREN ZUM BETRIEB SOLCH EINER AEROSOLBILDENDEN DÜSE
BUSE D'AÉROSOLISATION ET PROCÉDÉ DE FONCTIONNEMENT D'UNE TELLE BUSE D'AÉROSOLISATION

(43) Date of publication of application: 20.02.2019
(73) Proprietor: KAER Biotherapeutics Corporation, Escondido, CA 92029 (US)
(72) Inventor: YEATES, Donovan, B., Escondido, CA 92027 (US); HENG, Xin, Mckinney, TX 75070 (US)
(74) Representative: Schaumburg und Partner Patentanwälte mbB
(86) International application number: PCT/US2016/027804
(87) International publication number: WO 2017/180151

(56) References cited:
- WO-A1-2013/125555
- US-A- 2 152 767
- US-A1- 2005 196 345
- US-A1- 2012 318 259
- US-A1- 2014 332 601
- US-B2- 7 568 480
- US-B2- 8 375 987
- US-B2- 8 733 342

## Description

The generation of high concentrations of fine particle respirable aerosols from viscous solutions and solutions containing solutes of high molecular weight compounds and or liposomes have been fraught with difficulty, especially if these solutions have low surface tensions. Deep lung deposition is best achieved by inhaling particles having a mass median aerodynamic diameter (MMAD) of less than 4 µm. The present disclosure relates to a nozzle for creating liquid aerosols from solutions or suspensions including those of high viscosity and low surface tension together with the evaporation of the liquid from these aerosols to further reduce the particle size and to markedly increase the particle concentration in the suspending gas while reducing the total gas output to be more in balance with a desired delivery rate for particle collection, as well as their delivery to animals and humans.

Acute respiratory distress syndrome, ARDS, including acute lung injury, ALI, are of multifactorial etiology and afflict 190,000 Americans each year including 18% of all ventilated patients. Despite sophisticated intensive care, the mortality rate ranges between 15 and 72% with a mean of 43%. Patients with ARDS have impaired surfactant function and continued inflammation-induced inactivation of surfactant indicating that a single treatment with surfactant is likely inadequate. Surfactant delivery to patients with ARDS demonstrates that instillation of surfactant may improve oxygenation for ≥ 120 hours. However liquid bolus administration of surfactant results in a high rate of adverse effects and thus cannot be considered an effective adjunctive therapy in patients with acute lung injury. Multiple boluses of intrapulmonary instillations of surfactant are traumatic for the patient and physician alike and lead to undesirable peri- and posttreatment events. The delivery of surfactant in a gentler, non-invasive manner would empower physicians to provide improved life support and potentially a marked improvement in survival.

In the following, various prior art options for aerosol surfactant delivery are discussed. The delivery of pulmonary surfactant to treat lung diseases such as ARDS and respiratory distress syndrome, RDS, has been via the instillation of 20 to 80 mg/ml of suspensions of surfactant at doses generally ranging from 100 mg to 200 mg/kg or 100-700 ml for a 70 kg adult. Aerosolized surfactant may require a considerably lower dose (1/20) to achieve a similar physiological effect. It also eliminates the hypercapnia seen on bolus instillation. However, it is not yet part of the clinical armamentarium. This has been largely due to the difficulty of aerosolizing and delivering high concentrations of surfactant within a relatively short treatment time. In neonates, aerosolized surfactant was delivered over 3 hours. This situation is markedly exacerbated when attempting to deliver the same dose/kg of aerosolized surfactant to adults.

Mesh-type nebulizers generate soft mists through the creation of aerosols formed by liquids passing through an array of 2-3 µm diameter holes in a mesh. The energy to produce the aerosol is provided by electromechanical vibration of the mesh, or a horn located behind the mesh. A major drawback, however, is that the output of these nebulizers is limited by the viscosity of the nebulized formulation. Using low molecular weight polyols, it was demonstrated that the output markedly decreased or ceased when the viscosity was greater than 2 cSt (~2 cSt). The output of surfactant from one such nebulizer known by the trade name Aeroneb has been reported to aerosolize 0.1 mllmin resulting in the delivery of just 3 mg/min of surfactant at its output.

While jet type atomizers can aerosolize more viscous solutions their output is generally reduced to about 0.3 ml/minute or less for particles sizes of about 3 µm mass median aerodynamic diameter, MMAD. Aerosol outputs of surfactant of up to 7 mg/min have been reported by using these jet type atomizers. In addition, jet type atomizers typically recirculate the fluid that is not presented at the output leading to increasing concentrations of the solute, sol or liposomal content resulting in increased viscosity, decreased output and a change in the particle size. In addition, foam created in by surface active agents in the atomizer can also dramatically inhibit aerosol formation.

A capillary aerosol generator has been reported to be used by Discovery Laboratories to deliver surfactant to neonates. In this generator the surfactant suspension is heated in a capillary tube and evaporated. The output aerosol comprises the particles condensed on nuclei when the vapor is cooled. Such devices have the potential to cause heat-induced surfactant degradation. This device can take over an hour to produce 100 mg of surfactant in aerosol form.

Recently surfactant has been reported to be delivered from dispersing a dry powder of surfactant. Delivery rates of 6.6 mg/min in 0.84 liters of air have been reported. However, the efficiency of the delivery system has not been reported and is considered likely very low.

From the United States patent US 9,120,109 B2 aerosol generation from a nozzle is known comprising a fluid supply through a fluid supply orifice in close proximity to a plate, with the plate having an orifice of the same size as the fluid supply orifice. This proximity of the fluid supply orifice to the plate orifice was 0.1 to 0.25 times the diameter of these orifices. This patent further describes a transition from focused-flow (FF) to turbulent-flow-focusing (TFF) to flow-blurring (FB) and describes an observed reduction in particle size as the Reynolds number increases to what is likely a fully developed turbulent flow.

The aforementioned prior art technologies have not been shown to provide clinically relevant doses of surfactant to the adult respiratory tract, let alone to deliver a high mass in a short treatment time targeted at alveolar deposition. Therefore, these prior art technologies fall short on delivering certain drugs at the required delivery rate, for instance to treat ARDS and other respiratory diseases whose efficacy may depend on higher doses being delivered to the lungs in a shorter time span.

From the international patent application publication WO 2013/125555 A1 a nozzle for generating a spray is known, specifically for atomizing a liquid without using a power supply and with low energy. The suggested use is in the fields of medical devices (for example inhalers), cosmetic drug solution sprayers, moisturizing drug solution sprayers.

It is an object of the invention to create a nozzle and a corresponding method of operating such a nozzle that enables efficient generation of an aerosol comprising small particles from fluids, including those of relatively high viscosities, and/or with low surface tensions, at a high aerosolization rates with a relatively narrow particle size distributions.

US 2 152 767 discloses a spray gun in which material is atomized by means of compressed air and is ejected from a tubular nozzle.

It is an object of the invention to avoid any formation of larger droplets dripping from an outer surface of the nozzle, specifically when aerosolizing fluids of higher viscosity and at a high aerosolization rate.

Additional objects of the invention become apparent from the advantages achieved and described below.

These and other objects are achieved by a nozzle with the features claimed in claim 1 . Advantageous further embodiments are claimed in the dependent claims.

Preferably two or more circumferentially spaced pressurized gas supply channels lead to a pressure equalization chamber, and a circumferential converging annular channel at an acute angle with respect to the central axis that has a pressurized gas exit
opening in close proximity to the fluid exit orifice so that at least a central portion of this pressurized gas interacts with fluid exiting from the fluid exit orifice and a peripheral portion of this gas exits the aerosol exit orifice without substantial interaction with the liquid, especially when the aerosol exit diameter is 1.5 times larger than the diameter of the fluid exit orifice. Of note, the configuration of this preferred embodiment is designed for maintaining the patency of the converging annular pressurized gas channel by the elimination of "dead volumes" wherein fluid may accumulate and potentially result in nozzle malfunction.

Preferably, an aerosol exit channel length of the aerosol exit channel is less than the aerosol exit orifice diameter. A comparatively short aerosol exit channel facilitates the use of the nozzle over a wide range of pressurized gas, especially those pressures above 103 kPa (15 psi) and also minimizes the collection of any fluid particles on the exit channel.

As a particularly surprising effect of the incident invention, the aerosol formed within the nozzle exits through an aerosol exit orifice within a central core surrounded by a sheath of gas largely, if not entirely, devoid on particles. This sheath helps prevent droplets depositing on the outer surface of the nozzle that may cause the dripping of fluid.

The fluid to be aerosolized, may be a medication having a fluid viscosity which may be as high as 100 cSt and that may be aerosolized even at rates between 100 µl and 3 ml/minute, thereby delivering masses of a medication from 30 to 285 mg/minute with a fluid particle size having a mass median aerodynamic diameter (MMAD) of 6 µm or less.

The invention enables the generation of fine particle aerosols from solutions with viscosities up to at least 40 cSt with a relatively narrow size distribution (σ_{g} of less than 2.4 and in many cases less than 2). Another advantage of the incident invention is that the liquid aerosol is prevented from contacting the gas confining aerosol exit orifice, as the liquid aerosols are generated with a gas sheath between the stream of aerosol generated and the perimeter of the aerosol exit nozzle. This results in a generation of aerosols by a single pass nozzle with 100% aerosol generation efficiency. Consistent with this 100% aerosol generation efficiency, aerosols can be generated over a period of at least 8 hours without substantial solute build-up on the nozzle.

The incident invention further accomplishes generating fine particle aerosols with relatively low pressures from solutions, and solutions of liposomes and sols.

By combining the incident invention with additional device elements, the particle size can be further reduced by the evaporation of the solvent from the aerosols generated by the nozzle through dilution of the droplet aerosol with a dilution gas followed by increasing the particle concentration of these aerosols using a virtual impactor. This results in a smaller diameter dry powder aerosol in a reduced gas volume.

The incident invention further accomplishes delivering these aerosols at flow rates between 10 l/min to 200 l/min and delivering fine particles aerosols of surface active agents at rates of over 100 mg/min.

According to an embodiment of the invention, in this nozzle for generating fine particles aerosols from a solution or suspension, a fluid to be aerosolized is fed by means of a pump through a channel of length 1.5 mm and 250 µm in diameter within a first cone-like structure having an acute conical wall angle of 17 degrees from its axis and tapered towards the output of the channel contained therein, hereinafter referred to as a fluid exit orifice. Coaxial with this channel is an aerosol exit orifice that is larger than that of the fluid exit orifice and in a preferred configuration separated by a distance of 0.5 mm from the entrance to an aerosol exit channel. In a preferred arrangement, the diameter of the aerosol exit orifice is between 1.5 and 4 times larger than the diameter of the fluid exit orifice and hereinafter referred to as an aerosol exit orifice. At its circumference, in a preferred configuration, the aerosol exit channel length through which this aerosol exit orifice penetrates is just 180 µm. An external surface surrounding this aerosol exit orifice is of convex parabolic nature such that eddies in the gas flow around the aerosol exit orifice are minimized as is any particle deposition on or near this aerosol exit orifice. A pressurized gas exit is coaxially centered in a second cone-like structure at the apex of which is the aerosol exit channel. Preferably, the converging annular pressurized gas channel comprises a first partial annular channel and a second partial annular channel, the first partial annular channel has inner and outer walls formed by a conical wall and an inner front insert wall along which the pressurized gas flows with the acute flow angle being determined by a conical wall angle and a front insert wall angle with respect to the central axis in the area of the fluid exit orifice. The circumferential walls of this second cone-like structure leading to this pressurized gas exit encloses preferably an acute angle of 35 degrees adjacent to the nozzle, i.e. the angle between the central axis and the circumferential cone surface is about 17.5 degrees, such that there is a circumferential converging gas channel between the inner first cone-like structure and the second cone-like structure.

According to the invention, the fluid supply channel is provided within a cone-shaped fluid supply nozzle insert forming the conical wall that is the inner wall of the first partial annular channel, designed as a converging circumferential compressed gas channel that is axially centered in a cone shaped hollow space of the front insert forming the inner front insert wall that is the outer wall of the first partial annular channel.

Compressed gas enters two ports in a nozzle holder barrel and in a preferred configuration flows through two 1.2 mm diameter channels along the nozzle holder barrel. The channels terminate in a small annular chamber which enables equalization of the gas pressure and thus uniform flow of this gas between the fluid cone and the second cone-like structure such that the central portion of the gas interacts with the fluid as it exits from the fluid exit orifice. This fluid is aerosolized within the aerosolizing space between the fluid exit orifice and the entrance to the aerosol exit channel so that the aerosol exits the aerosol exit orifice as a column-like stream of aerosol particles surrounded by a sheath of gas at the aerosol exit orifice that is devoid of particles. An exterior surface of the exit orifice is convex to prevent eddies and particle deposition surrounding the aerosol exit orifice.

According to a preferred embodiment for generating a dry powder aerosol, this column of aerosol particles enters an evaporation chamber. A coaxial counter-flow jet of gas arrests the plume about half-way between a counter-flow output port and the aerosol exit orifice. This aerosol is transported along a quartz evaporation chamber by a dilution gas to a virtual impactor. The compressed gas for the nozzle and counter-flow gas as well as the dilution gas can be heated to enhance the evaporation of the fluid from the particles. In addition, to enhance evaporation of the fluid for the aerosol droplets there is an infrared bulb located adjacent to the evaporation chamber and an infrared reflector on the opposite side of the evaporation chamber. The evaporation chamber is comprised of a quartz tube as quartz is transparent to infrared radiation. The rate and degree of fluid evaporation is controlled by the temperatures and flow-rates of the gasses as well as the intensity of the infrared radiation. The resultant aerosol is accelerated by acceleration nozzles in the virtual impactor. The momentum imparted to these particles carries them across a small gap where most of the gas escapes the aerosol mutually perpendicular to the gas flow and most of the aerosol particles enter aligned deceleration nozzles on the other side of the gap. The aerosol exiting the deceleration nozzles flows through an output cone.

According to the invention, the aerosol exit orifice diameter is at least 1.3 times larger than the fluid exit orifice diameter. Other variations are possible, for instance aerosol exit orifice diameter may be 1.3 - 5 times larger than the fluid exit orifice diameter.

According to the invention, the distance between the fluid exit orifice and the entrance to the aerosol exit channel defining a length of the aerosolizing space is between 100 µm and 2 mm. More particularly, the length of the aerosolizing space may be between 200 µm and 1 mm. The aerosol is formed within the aerosolizing space that is provided within the nozzle, i.e. the aerosol forms substantially within the nozzle. As a more particular example, a length of the aerosolizing space of 500 µm has proven to be effective. According to another preferred embodiment, at least 2 pressurized gas supply channels are circumferentially spaced with respect to each other and extend to a pressure equalization chamber. It is possible to have more than two circumferentially spaced pressurized gas supply channels, for instance 6 or more around the circumference, depending on how much space is available for accommodating circumferentially spaced pressurized gas supply channels of sufficient cross-section.

In a preferred embodiment the nozzle comprises a barrel holding the cone-shaped fluid supply insert which incorporates the fluid exit orifice. The front insert forms the outer nozzle surface, and at least part of the cone shaped hollow space forms the outer wall of the first partial annular channel of the at least one converging annular pressurized gas channel. This design has the converging annular channel that has cone-shaped inner and outer walls. This design is manufacturing and assembly friendly and provides a good sized cross-section of the converging annular channel that can be easily adjusted by adjusting the inner diameter of the cone shaped hollow space of the front insert and/or the outer diameter of the cone-shaped fluid supply nozzle insert. This way, a preferable divergence of the first partial annular channel, for instance at an acute divergence angle between 1 and 30°, can be established, the acute divergence angle being the difference between the conical wall angle and the front insert wall angle. This divergence helps prevent a back flow of the fluid, specifically when the front insert wall and the conical wall are made hydrophobic, omniphobic or lyophobic.

According to another preferred embodiment, the outer nozzle surface area is convex in the area of the aerosol exit orifice. Specifically, it is preferred if the aerosol exit orifice has a substantially sharp edge with respect to the outer nozzle surface through which the aerosol exit orifice opens. Further, it is preferred if at least the outer nozzle surface area around the aerosol exit orifice is not recessed, i.e. the aerosol exit orifice is not provided within a dimple in the outer nozzle surface. A comparatively short aerosol exit channel length helps to enable higher aerosol exit orifice gas flows and avoids any accumulation of droplets or solid material around the aerosol exit orifice and any ensuing dripping.

Preferably, the aerosolizing space has the shape of a truncated cone having its small diameter at an entrance to the aerosol exit channel. The aerosol exit channel is very short, for instance substantially shorter than the diameter of the aerosol exit orifice.

Preferably, the inner and outer walls and the outer nozzle surface area are hydrophobic, omniphobic, or lyophobic.

According to a preferred embodiment, the aerosol exit orifice has a diameter between 400 µm and 1.2 mm. A goal is to avoid a focused flow with a Raleigh breakup in the cone-shaped plume at a distance from the aerosol exit orifice, but rather, according to the invention, to create a more column shaped aerosol plume that is essentially formed already within the nozzle in the aerosolizing space. Specifically, diameters between 500 µm and 1 mm have turned out to be advantageous, but any diameter works that is large enough to avoid the focused flow as known in the prior art but at the same time defines an aerosolizing space within the nozzle so that the aerosol is predominantly formed within the nozzle and exits as an aerosol through the aerosol exit orifice.

According to a preferred embodiment, the acute angle is between 10 and 60°.

Specifically advantageous is an angle of between 10 and 45°, more preferably between 13 and 30°. Additionally, the pressure impeding fluid flow between two converging lyophobic surfaces at an acute angle must increase as the surfaces approach each other. Thus, in the nozzle this impedes or prevents fluid back flow in the converging annular pressurized gas channel to the pressure equalization chamber and reduces or eliminates any resultant untoward effects on the aerosolization mechanisms.

According to a preferred embodiment of the nozzle, the nozzle is configured to form the aerosol within the aerosolizing space and exiting this aerosolizing space by the aerosol within a sheath of gas largely devoid of any aerosol particles. This also refers to the preferred column shaped plume with the particle-free sheath around the plume at the aerosol exit orifice avoiding particles impacting with a nozzle surface and accumulating or dripping from that surface.

According to another preferred embodiment of the nozzle , the nozzle is configured to generate the aerosol particles having a particle size at a given gas pressure that is smaller than generating these particles from a smaller orifice under conditions of focused flow.

Preferably, the size of the particles generated is substantially inviscid for fluid viscosities less than 40 cSt, i.e. the influence of the viscosity on the particle size is independent from the viscosity - apart from a minor influence that may for many practical purposes be ignored.

In a preferred embodiment, the fluid to be aerosolized is a surfactant suspension, particularly for the treatment of ARDS as high surfactant delivery rates are required.

According to a preferred embodiment, the fluid to be aerosolized is a surfactant suspension that mimics lung surfactant.

According to a preferred embodiment, the nozzle is further configured to generate fine particle aerosols from solutions with viscosities greater than 40 cSt.

According to a preferred embodiment, the nozzle is further configured to generate fine particle aerosols from aqueous suspensions with low surface tensions.

According to a preferred embodiment, the nozzle further provides the surfaces of the converging annular channel and pressure equalization chamber with hydrophobic, omniphobic or lyophobic properties to avoid wetting of these surfaces by solution or suspension residues.

According to a preferred embodiment, the nozzle is configured to impede or prevent back flow of the aerosolizing fluid and potential nozzle malfunction by choosing an appropriate acute divergence angle and by making the inner front insert wall and the conical wall of the first partial annular channel hydrophobic, omniphobic or lyophobic.

### BRIEF DESCRIPTION OF THE DRAWINGS

FIG. 1 shows a perspective view of an embodiment of a nozzle according to the invention assembled to a nozzle holder.
FIG. 2 shows an exploded perspective view of the nozzle and nozzle holder shown in FIG. 1.
FIG. 3 shows a side view of the nozzle and nozzle holder shown in FIG. 1.
FIG. 4 shows a sectional view of the nozzle and nozzle holder along the plane A-A as indicated in FIG. 3.
FIG. 5 shows a detail sectional view of the nozzle and nozzle holder of the detail B as indicated FIG. 4.
FIG. 6 shows a side view of a front insert of the aerosol exit nozzle shown in FIGs. 1-5.
FIG. 7 shows a sectional view of the front insert along the plane C-C as indicated in FIG. 6.
FIG. 8 shows a side view of a cone-shaped fluid supply nozzle insert of the nozzle shown in FIGs. 1-5.
FIG. 9 shows a sectional view of the cone-shaped fluid supply nozzle insert along the plane E-E as indicated in FIG. 8.
FIG. 10 shows the nozzle and nozzle holder placed in a flow distributer and an aerosol processing system used in conjunction with the nozzle according to the nozzle shown in FIGs. 1-5.
FIGs. 11A-11B shows that the aerosol plume from a nozzle with an aerosol exit orifice of 300 µm in diameter comprises a central fluid stream that breaks into droplets outside of the nozzle at compressed air pressures of both 207 KPa (30 psi) (FIG. 11A) and 414 KPa (60 psi) (FIG. 11B).
FIGs. 12A-12B shows that the aerosol plume from a nozzle with an aerosol exit orifice of 700 µm in diameter shows that the fluid is aerosolized within the nozzle and forms a columnar like aerosol plume at compressed air pressures of both 207 KPa (30 psi) (FIG. 12A) and 414 KPa (60 psi) (FIG. 12B).
FIG. 13 shows the aerosolization plume that is generated by a nozzle with an aerosol exit orifice of 1200 µm in diameter at a compressed air pressure of 414 KPa (60 psi).
FIG. 14 shows a diagram demonstrating the influence of the aerosol exit orifice diameter (AEOD) on the mass median aerodynamic diameter (MMAD) of the aerosol particles in µm.
FIG. 15 shows a diagram demonstrating the influence of the compressed air pressure (CAP) in psi on the mass median aerodynamic diameter (MMAD) of the aerosol particles in µm.
FIG. 16 shows a diagram demonstrating the influence of the kinematic viscosity KV of the fluid to be aerosolized in cSt on the mass median aerodynamic diameter (MMAD) of the aerosol particles in µm.
FIG. 17 shows a diagram demonstrating the influence of the fluid flow rate (FFD) of the fluid to be aerosolized in ml/min on the mass median aerodynamic diameter (MMAD) of the aerosol particles in µm for both PVP solutions and surfactant suspensions.

### DETAILED DESCRIPTION OF THE DRAWINGS

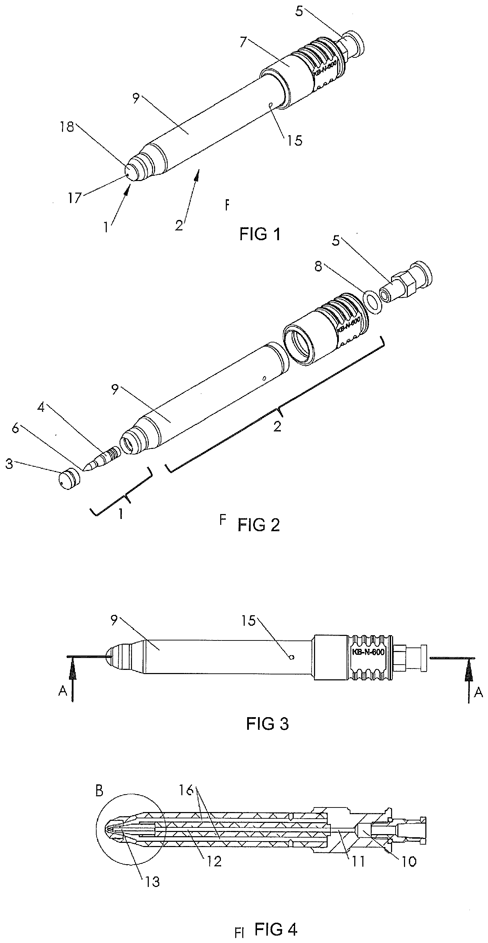

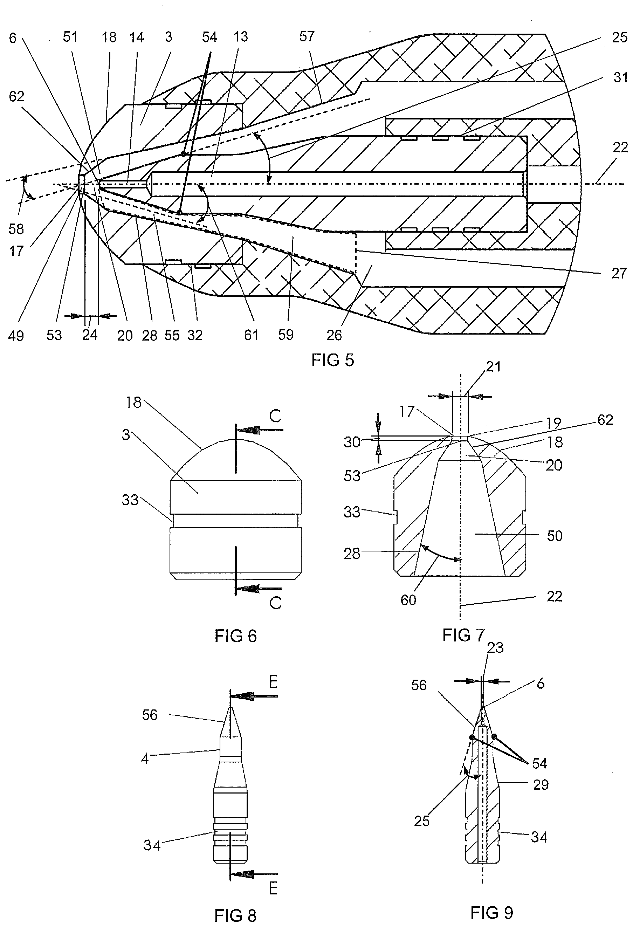

Referring to FIGs. 1 - 9 a nozzle 1 according to the incident invention that is mounted to a nozzle holder 2 is shown. In a preferred configuration these parts are made of ULTEM^{™}resin, albeit other materials may be used. As best seen in FIG. 2, the nozzle 1 includes a front insert 3 and a cone-shaped fluid supply nozzle insert 4. A fluid to be aerosolized is fed under pressure into a Luer connector 5 that is connected to a knob 7 on the nozzle holder 2. At the front end of the nozzle 1, specifically according to this embodiment in the front insert 3, the nozzle 1 comprises an aerosol exit orifice 17 located at an outer nozzle surface 18 having a circumferential edge 19 (see FIG. 7). There is an aerosolizing space 20 between a fluid exit orifice 6 (see FIGs. 5 and 9) and an entrance 53 to an aerosol exit channel 49 and bounded by an aerosolizing space wall 62 (see FIGs. 5 and 7). The aerosol exit orifice 17 has an aerosol exit orifice diameter 21 and a central axis 22.

As the fluid is fed under pressure through the Luer connector 5, an O-ring 8 prevents any fluid leakage between the Luer connector 5 and the knob 7. The nozzle holder 2 includes a barrel 9 such that the fluid, in a preferred configuration, flows through the Luer connector 5 to channel 10 and from there through channel 11 to channel 12 within the barrel 9 of the nozzle holder 2. This fluid then flows from channel 12 to channel 13 and into a very narrow fluid supply channel 14 that in a preferred configuration is 250 µm in diameter and 1.5 mm long but other diameters between 20 and 400 µm are also feasible options. At the end of the very narrow fluid supply channel 14 the fluid exit orifice 6 is provided, and having, according to this embodiment, the same fluid exit orifice diameter 23 as the very narrow channel 14.

Compressed gas enters the barrel 9 of the nozzle holder 2 through gas entry ports 15 on each side of the barrel 9. This gas flows into each of two pressurized gas supply channels 16 towards (see FIG. 4) the nozzle 1. According to one embodiment, the pressurized gas supply channel 16 leads to a pressure equalization chamber 26 and a converging annular pressurized gas channel 27 converging to an annular neck 54. The converging annular pressurized gas channel 27 comprises a first partial annular channel 55 extending between the annular neck 54 and the fluid exit orifice 6, while a second partial annular channel 59 extends substantially between the annular neck 54 and the pressure equalization chamber 26. Due to the annular neck 54, the second partial annular channel 59 also acts to uniform gas flow to the first partial annular channel 55. In the first partial annular channel 55 an inner front insert wall 28 and a conical wall 56 are diverging, at an acute divergence angle 58, from which the gas enters into the aerosolizing space 20 in the proximity of fluid orifice exit 6. The average between the conical wall angle 25 and the front insert wall angle 60 determined by the sum of the conical wall angle 25 and the front insert wall angle 60 divided by two and is hereinafter referred to in case of divergence simply as the acute gas flow angle 61 like in case of a non-diverging first partial annular channel 55.

The converging annular pressurized gas channel 27 is defined by the inner front insert wall 28 together with a converging wall 57 in the barrel 9 and an outer cone-shaped fluid supply nozzle insert wall 29. The first partial annular channel 55 is defined by the inner front insert wall 28 and conical wall 56 adjacent to the fluid exit orifice 6 extending at an acute conical wall angle 25 with respect to the central axis 22. Several of the pressurized gas supply channels 16 can be provided in a circumferentially spaced fashion with respect to each other, leading to the pressure equalization chamber 26, and the converging annular pressurized gas channel 27. Beneficial from a manufacturing and assembly point of view is an embodiment with the front insert 3 and cone-shaped fluid supply nozzle insert 4, the respective component parts being shown in FIGs. 6-9, where for the purpose of assembly, the barrel 9 of the nozzle holder 2 is provided with a first hole 31 into which the cone-shaped fluid supply nozzle insert 4 is inserted, and a second hole 32 into which the front insert 3 is inserted. By choosing appropriate tolerances for the holes 31 and 32 in relation to the outer diameters of the inserts 4 and 3, respectively, these may hold themselves in place by frictional forces. A more economic manufacturing method can be accomplished by gluing the inserts 3 and 4 into the respective corresponding holes 32 and 31, and for accommodating glue, respective recesses 33 and 34 can be provided in the respective inserts 3 and 4, as shown in FIGs. 6-9.

According to this embodiment, the gas flows from the pressurized gas supply channels 16 into the pressure equalization chamber 26 and from there into the converging annular pressurized gas channel 27 and into the first partial annular channel 55. At a distance 24 from the entrance 53 of the aerosol exit channel 49, the first partial annular channel 55 intersects the central axis 22 in the vicinity of the fluid exit orifice 6. A fluid jet exits through the fluid exit orifice 6 in direction of the central axis 22 where it interacts with the pressurized gas at a pressurized gas exit 51. According to a preferred embodiment, the distance 24 is 500 µm which distance marks approximately the length of the aerosolizing space 20. Other distances between 100 µm and 2 mm are possible. This exiting pressurized gas blasts the fluid exiting the fluid exit orifice 6 at the end of a fluid supply channel 14. As shown in FIG. 7, an aerosol exit channel length 30 within the front insert 3 is preferably small, for instance, according to a preferred embodiment, is just 180 µm. Fine particles are created as a result of this blast of high pressure air exit through the aerosolizing space 20 and the aerosol exit orifice 17 to form an aerosol plume. The fine particles exit through a central core of the aerosol exit orifice 17 to form a column-like aerosol plume. Between the central aerosol core and the circumferential edge 19 of the aerosol exit orifice 17, a concentric particle free zone exists. The high velocity at which the aerosol and therefore the high velocity aerosol gas exits the aerosol exit orifice 17 generates a Venturi effect, entraining particle free gas sweeping over the outer nozzle surface 18. In addition to the concentric particle free zone, the gas entrainment induced by the Venturi effect helps ensure that this outer nozzle surface 18 remains free of particle deposition. Helping with this gas entrainment is the convex shape of the outer nozzle surface 18, preferably an aerodynamically optimized parabolic shape to increase a sweeping effect. These gas channels and chambers 16, 26, 27, 49 as well as the convex outer nozzle surface 18 can be treated to be hydrophobic, omniphobilic or lyophobic as a further precaution to maintain nozzle patency and function.

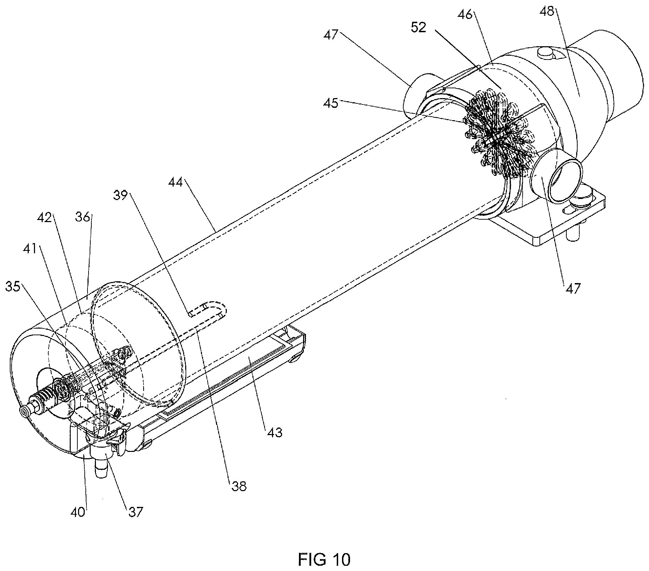

The nozzle according to the invention creates fluid particles (droplets) small enough to be directly inhaled for certain applications. However, a dry powder aerosol might be preferred. The incorporation of these nozzles according to the incident invention in an aerosol processing system shown in FIG. 10 facilitates the evaporation of solvents containing the medication, the reduction of the particle size and enables an increase in the concentration of the number of particles. This dry powder aerosol generating apparatus and several components thereof are described in the United States Patents 8,596,268; 8,616,532; 8,375,987.

In this arrangement, during operation, the barrel 9 of nozzle holder 2 is inserted into a channel 35 in a flow distributer 36. Compressed gas enters port 37. Within the flow distributer 36, the compressed gas is split into two streams. The first gas flow enters a circumferential grove whose position coincides with the location of the gas entry ports 15 on the barrel 9 of the nozzle holder 2. The second gas flow is constrained by an orifice within the flow distributer 36. The second gas flow flows into a counter-flow tube 38. The orifice diameter in this second flow path is adjusted such that the counter-flow gas arrests the aerosol plume roughly midway between the aerosol exit orifice 17 and an output port 39 of the counter-flow tube 38. The fluid is evaporated from the aerosol particles by the provision of heated gas to both the gas entry ports 15 and the counter-flow tube 38. In addition, a third flow of warmed gas enters port 40 which is conditioned between two baffles 41 and 42. This gas then surrounds the nozzle barrel 9 and dilutes the aerosol emitted from aerosol exit orifice 17. Evaporation is aided by infrared radiation from an infrared source 43. The aerosol transits through a quartz evaporation tube 44 and enters acceleration nozzles 45 on a virtual impactor 46. Most of the gas exits through two exhaust ports 47. Most of the particles cross a gap between the acceleration nozzles 45 and radially aligned deceleration nozzles 52. The concentrated aerosol exits the virtual impactor 46 into an output cone 48.

### EXAMPLES

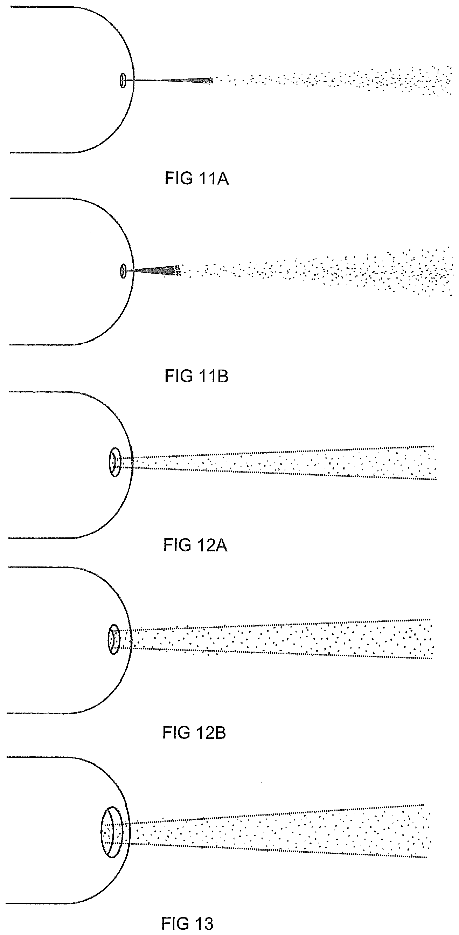

To evaluate the nature of a remarkable decrease in particle size with increasing aerosol exit orifice diameter 21, a series of photographs of aerosol plumes generated by these nozzles were taken while generating aerosols of water. Exemplary aerosol exit orifice diameter 21 variations are shown in FIGs. 11A and 11B with an aerosol exit orifice diameter 21 of 300 µm; FIGs. 12A and 12B with an aerosol exit orifice diameter 21 of 700 µm, and FIG. 13 with an aerosol exit orifice diameter 21 of 1200 µm. The fluid exit orifice diameter 23 in all cases was 250 µm and the distance 24 between the fluid exit orifice 6 and the entrance 53 to the aerosol exit channel 49, i.e. the length as the aerosolizing space 20 was 500 to 520 µm and the aerosol exit channel length 30 was 180-220 µm in all examples shown in FIGs. 11-13. The transformation of the nature of aerosol formation and emission from the nozzles is illustrated in FIG 11A-B by comparing the plume formed outside a 300 µm diameter aerosol exit orifice 17 at 207 kPa (30 psi) and 1 ml/min and 414 kPa (60 psi) at 3 ml/min with the aerosol plume formed in the example shown in FIG. 12A-B within the aerosolizing space 20 exiting through a 700 µm diameter aerosol exit orifice 17 operated at 207 kPa (30 psi) and 1 ml/min and 414 kPa (60 psi) at 3 ml/min. The focused flow nature of the fluid as it exits the 300 µm diameter aerosol exit orifice 17 can be seen. This fluid stream from the 300 µm diameter aerosol exit orifice 17 undergoes Raleigh breakup to form a cone-shaped aerosol plume. In contrast, when a 700 µm diameter aerosol exit orifice 17 was used under the same compressed air pressures and fluid flow rates it can be seen that the aerosolization occurs prior to the aerosol exiting the aerosol exit orifice 17. It can also be seen that the plume is columnar shaped and that this columnar shaped plume exits the aerosol exit orifice 17 in a central core region of the nozzle surrounded by what appears to be a particle free zone between this central core region and the perimeter of the aerosol exit orifice 17. This particle-free gas surrounding the columnar aerosol plume can be seen in the examples shown in FIGs. 12A and 12B and even more clearly observed with the aerosol exit orifice 17 of 1200 µm in diameter (FIG 13), operated at 414 kPa (60 psi) with an aerosolization rate of 3 ml/min. The transition from the focused flow nature of the aerosol produced to a substantially columnar aerosol plume (FIG. 12A, 12B and 13) originating within the nozzle from the aerosolizing space 20 occurred for example at compressed air pressures above 276 kPa (40 psi) for a 400 µm diameter nozzle. This change in the nature of this aerosolization process was observed with all the larger nozzles and at all pressures tested. It was also observed that the aerosol free zone between the central columnar plume aerosol exit orifice increased with aerosol exit orifice diameter 21 and that the outer nozzle surface 18 of the aerosol exit orifice 17 was free from deposition of any fluid from the aerosol generated.

The performance of these nozzles was evaluated in conjunction with the aerosol processing system shown in FIG. 10 incorporated into a SUPRAER^{™} console. To evaluate the performance of these nozzles, aerosols were generated with aqueous solutions (w/v) of the excipient, polyvinylpyrrolidone (PVP) 8 kDa, 29 kDa, 40 kDa and 58 kDa as a protein, biologic or drug surrogate. The warmed compressed air and dilution air together with the infrared radiation were set to ensure complete evaporation of the water from the droplets generated, yielding dry aerosol particles. Measurements of particle size distributions at the output of the aerosol processing system were determined using a Marple-Miller cascade impactor. Measurements of the mass distributions were facilitated by the addition of 4% Allura Red AC (Sigma-Aldrich, USA) to the mass of PVP in the solution and analyzed spectrographically. The MMAD and the geometric standard deviation were determined from the cumulative log-probability plots. The outputs (delivered dose) of SUPRAER^{™} for nozzles were measured gravimetrically. The output was sampled using low resistance electrostatic filters at 44 l/min.

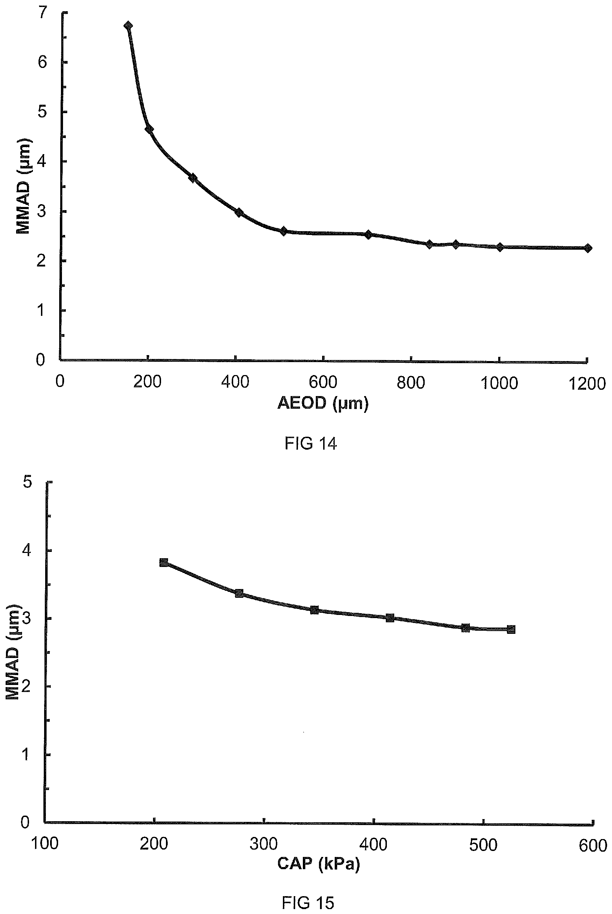

The effect of the aerosol exit orifice diameter 21 on particle size is shown in FIG. 14. At 207 kPa (30 psi) compressed air pressure at an aerosolizing rate of 1 ml/min and a concentration of 5% solution of 8 kDa PVP, the particle size of the output aerosol decreased with increases in the aerosol exit orifice diameter 21. The geometric standard deviation of the particle size distributions ranged from 1.7 to 2.15.

The diameter of the original droplets can be roughly estimated by multiplying the dry particle diameter by cube root of the ratio of water to PVP.

The effect of compressed air pressure on particle size is shown in FIG. 15. The output particle size as given by the MMAD decreases with increasing compressed air pressure. The particles were generated at 1 ml/min with a nozzle having an aerosol exit orifice diameter 21 of 700 µm and using a 10% solution of 8kDa PVP. Also, at 414 kPa (60 psi) aerosols of 40% 8kDa PVP generated at 1 ml/min had a MMAD of 5.1 µm and geometric standard deviation of 2.0 was observed.

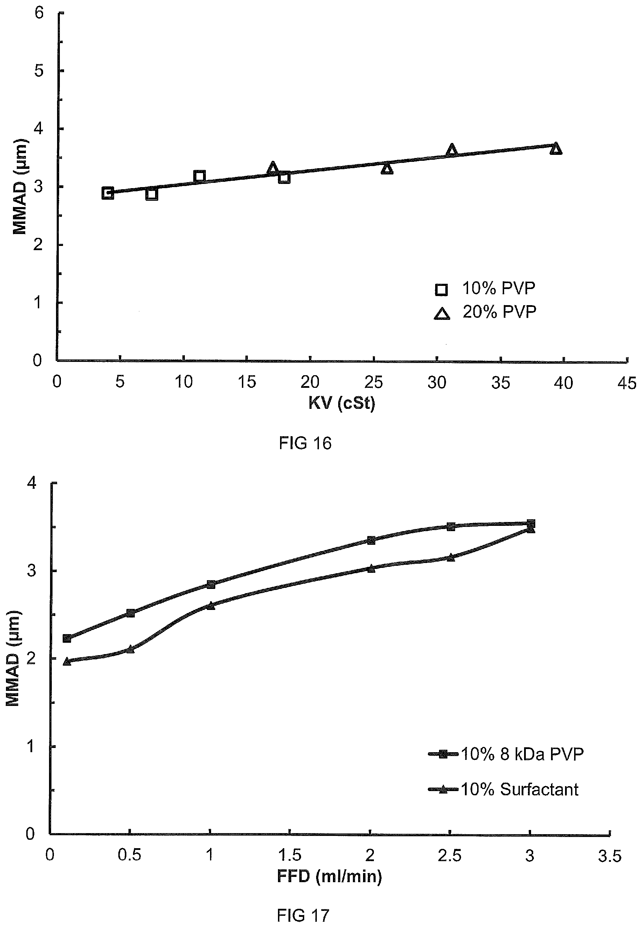

FIG. 16 demonstrates the effect of molecular weight/viscosity on the MMAD of the particles generated. The MMAD for 10% and 20% PVP solutions of 8kDa, 29kDa, 40 kDa and 58 kDa are shown. The particles generated from 20% PVP solutions, have a larger particle size than those generated from 10% PVP solutions. This FIG. 16 demonstrates that the particle size of the aerosols generated was relatively independent of the viscosity of the solution being aerosolized over the range of 4 to 40 cSt (~ 4 to 40 cP). This demonstrates an advantage of the incident invention, namely that the nozzle according to the incident invention delivers respirable aerosols over a wide range of viscosities at about the same particle size, i.e. is inviscid.

Aerosolization of solution concentrations of 5%, 10% 25% and 40% 8kDa, PVP with the 700 µm diameter aerosol exit orifice 17 produced particles with MMAD's of 2, 3, 3.2, and 4.2 µm, respectively, with geometric standard deviations between 1.6 and 2.0.

The nozzles and aerosol processing system were used to generate aerosols from surfactant suspensions, including those containing phospholipids mimicking pulmonary surfactants. Aerosols were generated at 1 ml/min from a 10% suspension of surfactant liposomes having a viscosity of 34 cP and surface tension in the 22-24 mN/m range.

FIG. 17 demonstrates the effect of solution flow rate on the particle size for the 700 µm diameter aerosol exit orifice 17 at 414 kPa (60 psi). There was a modest linear in particle size with 10% 8kDa PVP solution flow rates from 0.1 to 3 ml/min. When a 10% suspension of surfactant was aerosolized the particle size increased from 2.0 to 3.5 µm as the flow rate increased from 0.1 to 3 ml/min.

Table 1 demonstrates the mass output rate and efficiency for aerosols generated at increased flow rates for 20% 8kDa PVP and 10% suspension of surfactant using a 500 and the 700 µm diameter aerosol exit orifices 17, respectively.

**Table 1: The PVP mass output rate and efficiency for PVP and surfactant aerosols.**

| Nozzle AEOD (µm) | Solution | Pressure (kPa) | Solute Flow Rate (ml/min) | Output (mg/min) | Efficiency % |
|---|---|---|---|---|---|
| 500 | 20% 8 kDa PVP | 414 | 1 | 106 | 53% |
| | | | 2 | 252 | 63% |
| 700 | 10% Surfactant | 207 | 1 | 53 | 53% |
| | | 414 | 1 | 46 | 46% |
| | | | 2 | 92 | 46% |
| | | | 3 | 126 | 42% |

Another 8.8% surfactant sample that had a viscosity of 57cP measured using a capillary rheometer was tested. Using the 500 µm diameter nozzle at 414 kPa (60 psi) at an aerosolization rate of 2.5 ml/min and a MMAD of ~3.4 µm, an aerosol with a 1.7 geometric standard deviation was generated. A surfactant aerosol at a mass flow rate of 98 mg/min was collected.

Through the choice of aerosol exit orifice diameter 21, compressed air pressure, as well as solution concentration and solution flow rate, a selection of particles sizes and output rates over a wide range of concentrations and viscosities can be achieved. Of note, as the flow of fluid to be aerosolized is metered by a pump, the aerosol generation and output is constant. High concentrations (over 1 g/m³) of pure fine particle aerosols can be delivered, thus potentially reducing treatment times from 20-100 min to 5-10 minutes as well as reducing exposure to excipients often used in dry powder inhalers. Such a device offers an attractive alternative to mesh-type and jet-type nebulizers for the generation and delivery of aerosols from relatively viscous solutions including biologics and surface-active agents. Such a device facilitates the efficient generation of aerosols of selectable particle size and concentration.

The medical applications of the incident invention include, but are not limited to, the aerosol delivery to the respiratory tract of surfactant, liposomal formulations, antiinfectives, mucolytics and gene transfer agents having viscous and surface properties within the ranges herein described.

The applications of this incident invention extend beyond the delivery of medications to the generation of fine particle aerosols for consumer and industrial applications where the fluid to be aerosolized has a viscosity above 10 cSt and or a low surface tension.

### List of Reference Numerals

nozzle 1
nozzle holder 2
front insert 3
cone-shaped fluid supply nozzle insert 4
Luer connector 5
fluid exit orifice 6
knob 7
O-ring 8
barrel 9
channel 10
channel 11
channel 12
channel 13
fluid supply channel 14
gas entry port 15
pressurized gas supply channel 16
aerosol exit orifice 17
outer nozzle surface 18
circumferential edge 19
aerosolizing space 20
aerosol exit orifice diameter 21
central axis 22
fluid exit orifice diameter 23
distance 24
conical wall angle 25
pressure equalization chamber 26
converging annular pressurized gas channel 27
inner front insert wall 28
outer cone-shaped fluid supply nozzle insert wall 29
aerosol exit channel length 30
first hole 31
second hole 32
recess 33
recess 34
channel 35
flow distributor 36
port 37
counter-flow tube 38
output port 39
port 40
baffle 41
baffle 42
infrared source 43
quartz evaporation tube 44
acceleration nozzles 45
virtual impactor 46
exhaust ports 47
output cone 48
aerosol exit channel 49
cone-shaped hollow space 50
pressurized gas exit 51
deceleration nozzles 52
entrance 53
annular neck 54
first partial annular channel 55
conical wall 56
converging wall 57
acute divergence angle 58
second partial annular channel 59
front insert wall angle 60
acute gas flow angle 61
aerosolizing space wall 62

## Claims

1. A nozzle (1) for generating from a fluid and a gas an aerosol comprising fluid particles, the nozzle (1) comprising:
an aerosol exit orifice (17) extending to an outer nozzle surface (18) of the nozzle, wherein the aerosol exit orifice (17) has an aerosol exit orifice diameter (21), a central axis (22) and has a circumferential edge (19) from which an aerosol exit channel (49) of the nozzle extends from the outer nozzle surface (18) toward an aerosolizing space (20) within the nozzle (1) connecting the aerosolizing space (20) with the outer nozzle surface (18);
a fluid supply channel (14) with a fluid exit orifice (6) that is aligned on the same central axis (22) as the aerosol exit orifice (17) and opens into the aerosolizing space (20) within the nozzle at a distance (24) from an entrance (53) from the aerosolizing space (20) to the aerosol exit channel (49), the aerosol exit orifice diameter (21) being at least 1.3 times larger than a fluid exit orifice diameter (23);
a converging annular pressurized gas channel (27) extending at an acute gas flow angle (61) with respect to the central axis (22), said converging annular pressurized gas channel (27) having a pressurized gas exit (51) at the fluid exit orifice (6) so that pressurized gas exiting through the pressurized gas exit (51) intersects with fluid exiting from the fluid exit orifice (6); wherein the converging annular pressurized gas channel (27) extends between a cone-shaped fluid supply nozzle insert (4) of the nozzle, said cone-shaped fluid supply nozzle insert (4) having a conical wall (56), and a front insert (3) of the nozzle, said front insert (3) having an inner front insert wall (28), wherein said converging annular pressurized gas channel (27) comprises a first partial annular channel (55) and a second partial annular channel (59), the first partial annular channel (55) having inner and outer walls formed by the conical wall (56) and the inner front insert wall (28) along which the pressurized gas flows with the acute gas flow angle (61) being defined by a conical wall angle (25) as measured between the conical wall (56) and the central axis (22) and a front insert wall angle (60) as measured between inner front insert wall (28) and the central axis (22) at the fluid exit orifice (6), wherein the acute gas flow angle (61) is the sum of the conical wall angle (25) and the front insert wall angle (60) divided by two;
the fluid supply channel (14) is provided within the cone-shaped fluid supply nozzle insert (4) forming the conical wall (56) that is the inner wall of the first partial annular channel (55), designed as a converging circumferential compressed gas channel that is axially centered in a cone-shaped hollow space (50) of the front insert (3) forming the inner front insert wall (28) that is the outer wall of the first partial annular channel (55); **characterized in that**
the acute gas flow angle (61) is between 10 and 30°; and
the distance (24) between the fluid exit orifice (6) and the entrance (53) to the aerosol exit channel (49) is greater than twice the fluid exit orifice diameter (23) and within a range between 100 µm and 2 mm.

2. The nozzle according to claim 1, wherein an aerosol exit channel length (30) of the aerosol exit channel (49) is less than the aerosol exit orifice diameter (21), and wherein the aerosol exit orifice diameter (21) is at least 1.5 times larger than the fluid exit orifice diameter (23).

3. The nozzle according to any one of the preceding claims, wherein an outer nozzle surface (18) area is convex at the aerosol exit orifice (17).

4. The nozzle according to any one of the preceding claims, wherein the aerosolizing space (20) has the shape of a truncated cone having its small diameter at the entrance (53) to the aerosol exit channel (49).

5. The nozzle according to any one of the preceding claims, wherein at least one of the inner front insert wall (28), an outer cone-shaped fluid supply nozzle insert wall (29) of the cone-shaped fluid supply nozzle insert (4), aerosol exit channel (49), and an outer nozzle surface (18) area are hydrophobic, omniphobic, or lyophobic.

6. The nozzle according to any one of the preceding claims, wherein the first partial annular channel (55) diverges at an acute divergence angle (58) between 1 and 30°, the acute divergence angle (58) being the difference between the conical wall angle (25) and the front insert wall angle (60).

7. The nozzle according to claim 1, wherein the nozzle is configured to: aerosolize a fluid with a viscosity exceeding 4 cSt; and aerosolize the fluid at a rate of more than 1 ml/minute, thereby generating an aerosol containing a medication at a mass flow rate of at least 30 mg/minute in form of the fluid particles having a mass median aerodynamic diameter (MMAD) of 6 µm or less.

8. The nozzle according to claim 7, further configured to form the aerosol within the aerosolizing space (20) and exiting this aerosolizing space (20) by the aerosol encompassed within a sheath of gas largely devoid of any aerosol particles.

9. The nozzle according to claim 7 or 8, further configurerd to aerosolize a fluid with a viscosity between 4 and 40 cSt, wherein the mass median aerodynamic diameter (MMAD) of the particles generated in this viscosity range is up to 40% higher when the aerosol is generated from a fluid having a viscosity of 40 cSt versus a fluid having a viscosity of 4 cSt.

10. The nozzle according to any one of claims 7 - 9, further configured to aerosolize a fluid that is a surfactant suspension.

11. The nozzle according to any one of claims 7 - 10, further configured to generate particle aerosols from solutions with viscosities greater than 40 cSt, or comprising generating fine particle aerosols from aqueous suspensions with surface tensions of between 2 and 72 mN/m.

12. The nozzle according to any one of claims 7 - 11, configured to impede or prevent back flow of the aerosolizing fluid and potential nozzle malfunction by choosing an acute divergence angle (58) between 1 and 30° at which the first partial annular channel (55) diverges, said acute divergence angle (58) being the difference between the conical wall angle (25) and the front insert wall angle (60), and by making the inner front insert wall (28) and the conical wall (56) of the first partial annular channel (55) hydrophobic, omniphobic or lyophobic.

## Patentansprüche

1. Eine Düse (1), welche aus einer Flüssigkeit und einem Gas ein Aerosol erzeugt, welches Flüssigkeitströpfchen aufweist, wobei die Düse (1) versehen ist mit:
einer Aerosolaustrittsöffnung (17), welches sich zu einer äußeren Düsenoberfläche (18) der Düse erstreckt, wobei die Aerosolaustrittsöffnung (17) einen Aerosolaustrittsöffnungs-Durchmesser (21), eine Mittelachse (22), und eine Umfangskante (19) aufweist, von welcher aus sich ein Aerosolaustrittskanal (49) der Düse von der äußeren Düsenoberfläche (18) zu einem Aerosolisierungsraum (20) im Inneren der Düse (1) erstreckt, und so den Aerosolisierungsraum (20) mit der äußeren Düsenoberfläche (18) verbindet;
einem Flüssigkeitszuführungskanal (14) mit einer Flüssigkeitsaustrittsöffnung (6), welche zu der gleichen Mittelachse (22) wie die Aerosolaustrittsöffnung (17) ausgerichtet ist und zu dem Aerosolisierungsraum (20) im Inneren der Düse in einem Abstand (24) von einem Eingang (53) von dem Aerosolisierungsraum (20) in den Aerosolaustrittskanal (49) beanstandet ist, wobei der Aerosolaustrittsöffnungs-Durchmesser (21) wenigstens 1,3 mal größer als ein Flüssigkeitsaustrittsöffnungs-Durchmesser (23) ist;
einem konvergierenden Druckgas-Ringkanal (27), welcher sich in einem spitzen Gasflusswinkel (61) relativ zu der Mittelachse (22) erstreckt, wobei der konvergierenden Druckgas-Ringkanal (27) einen Druckgasausgang (51) bei der Flüssigkeitsaustrittsöffnung (6) aufweist, so dass das durch den Druckgasausgang (51) austretende, unter Druck stehendem Gas auf die aus der Flüssigkeitsaustrittsöffnung (6) austretende Flüssigkeit auftrifft; wobei sich der konvergierende Druckgas-Ringkanal (27) zwischen einem, mit einer konischen Wand (56) versehenen konusförmigen Flüssigkeitszuführungs-Einsatz (4) der Düse und einem Fronteinsatz (3) der Düse erstreckt, wobei der Fronteinsatz (3) eine innere Fronteinsatzwand (28) aufweist, wobei
der konvergierende Druckgas-Ringkanal (27) einen ersten ringförmigen Teilkanal (55) und einen zweiten ringförmigen Teilkanal (59) aufweist, wobei der erste ringförmige Teilkanal (55) innere und äußere Wände aufweist, welche durch die konische Wand (56) und die innere Fronteinsatzwand (28) ausgebildet sind, entlang welcher das Druckgas in dem spitzen Gasflusswinkel (61) strömt, welcher durch einen Konuswandwinkel (25) bestimmt wird, welcher zwischen der konischen Wand (56) und der Mittelachse (22) herrscht, und durch einen Fronteinsatzwandwinkel (60) bestimmt wird, welcher zwischen der innere Fronteinsatzwand (28) und der Mittelachse (22) bei der Flüssigkeitsaustrittsöffnung (6) herrscht, wobei der spitze Gasflusswinkel (61) die durch zwei geteilte Summe aus Konuswandwinkel (25) und Fronteinsatzwandwinkel (60) ist;
der Flüssigkeitszuführungskanal (14) im Inneren des konusförmigen Flüssigkeitszuführungs-Einsatzes (4) ausgebildet ist, welcher die konische Wand (56) aufweist, welche als Innenwand des ersten ringförmigen Teilkanals (55) fungiert, welcher als konvergierender Druckgas-Ringkanal ausgebildet ist, welcher achsgleich zu einem konischen Hohlraum (50) des Fronteinsatzes (3) ausgerichtet ist, welcher die innere Fronteinsatzwand (28) ausbildet, welche als die Außenwand des ersten ringförmigen Teilkanals (55) fungiert;
**dadurch gekennzeichnet, dass**
der spitze Gasflusswinkel (61) zwischen 10 und 30° liegt; und
der Abstand (24) zwischen der Flüssigkeitsaustrittsöffnung (6) und dem Eingang (53) in den Aerosolaustrittskanal (49) grösser als das Doppelte des Flüssigkeitsaustrittsöffnungs-Durchmessers (23) ist und in einem Bereich zwischen 100 µm und 2 mm liegt.

2. Die Düse nach Anspruch 1, wobei eine Aerosolaustrittskanallänge (30) des Aerosolaustrittskanals (49) kürzer als der Aerosolaustrittsöffnungs-Durchmesser (21) ist, und wobei der Aerosolaustrittsöffnungs-Durchmesser (21) wenigstens 1,5-mal größer als der Flüssigkeitsaustrittsöffnungs-Durchmesser (23) ist.

3. Die Düse nach einem der vorangehenden Ansprüche, wobei die äußere Düsenoberfläche (18) bei der Aerosolaustrittsöffnung (17) konvex ist.

4. Die Düse nach einem der vorangehenden Ansprüche, wobei der Aerosolisierungsraum (20) kegelstumpfförmig ausgebildet ist, wobei sich der kleinere Durchmesser am Eingang (53) in den Aerosolaustrittskanal (49) befindet.

5. Die Düse nach einem der vorangehenden Ansprüche, wobei wenigstens eine der inneren Fronteinsatzwand (28), einer äußeren, konusförmigen Flüssigkeitszuführungs-Einsatzwand (29) des konusförmigen Flüssigkeitszuführungs-Einsatzes (4) der Düse, des Aerosolaustrittskanals (49), und der äußeren Düsenoberfläche (18) hydrophob, omniphob oder lyphob ist.

6. Die Düse nach einem der vorangehenden Ansprüche, wobei der erste ringförmige Teilkanal (55) um einen spitzen Divergenzwinkel (58) divergiert, welcher zwischen 1 und 30° liegt, wobei der spitze Divergenzwinkel (58) die Differenz zwischen dem Konuswandwinkel (25) und dem Fronteinsatzwandwinkel (60) ist.

7. Die Düse nach Anspruch 1, wobei die Düse dazu ausgebildet ist, um:
eine Flüssigkeit mit einer 4 cST überschreitenden Viskosität zu aerosolieren; und
die Flüssigkeit mit einer Rate von mehr als 1 ml/min zu aerosolisieren, wodurch ein Medikamenten-Massenstrom von wenigstens 30 mg/min in Form von Flüssigkeitströpfchen mit einem medianen massenbezogenen aerodynamischen Durchmesser (MMAD) von 6 µm oder weniger erzeugt wird.

8. Die Düse nach Anspruch 7, welche weiter derart ausgebildet ist, dass das Aerosol in dem Aerosolisierungsraum (20) erzeugt wird und diesen Aerosolisierungsraum (20) mit einer Hülle aus Gas umschlossen verlässt, welche im Wesentlichen frei von Aerosol-Flüssigkeitströpfchen ist.

9. Die Düse nach Anspruch 7 oder 8, welche weiter dazu ausgebildet ist, eine Flüssigkeit mit einer Viskosität zwischen 4 und 40 cST zu aerosolisieren, wobei der
medianen massenbezogenen aerodynamischen Durchmesser (MMAD) der in diesem Viskositätsbereich erzeugten Flüssigkeitströpfchen bis zu 40 % höher ist, wenn das Aerosol aus einer Flüssigkeit mit einer Viskosität von 40cST im Vergleich zur einer Flüssigkeit mit einer Viskosität von4 cST erzeugt wird.

10. Die Düse nach einem der Ansprüche 7 - 9, welche weiter dazu ausgebildet ist, eine Flüssigkeit zu aerosolisieren, welche eine eine Tensidsuspension ist.

11. Die Düse nach einem der Ansprüche 7 - 10, welche weiter dazu ausgebildet ist, eine Flüssigkeit mit einer Viskosität von mehr als 40 cST zu aerosolisieren, oder Aerosole mit feinen Flüssigkeitströpfchen aus einer wässrigen Lösung zu erzeugen, welche eine Oberflächenspannung zwischen 2 und 72 Nm/m aufweisen.

12. Die Düse nach einem der Ansprüche 7 - 11, welche weiter dazu ausgebildet ist, einen Rückfluss von Aerosol-Flüssigkeit zu hemmen oder zu verhindern, um so eine Fehlfunktion der Düse zu vermeiden, indem ein spitzer Divergenzwinkel (58) zwischen 1 und 30° ausgewählt wird, um welchen der erste ringförmige Teilkanal (55) divergiert, wobei der spitze Divergenzwinkel (58) die Differenz zwischen dem Konuswandwinkel (25) und dem Fronteinsatzwandwinkel (60) ist, und indem die innere Fronteinsatzwand (28) und die konische Wand (56) des ersten ringförmigen Teilkanals (55) hydrophob, omniphob oder lyphob ausgebildet sind.

## Revendications

1. Buse (1) pour générer, à partir d'un fluide et d'un gaz, un aérosol comprenant des particules de fluide, la buse (1) comprenant :
un orifice de sortie d'aérosol (17) s'étendant vers une surface de buse extérieure (18) de la buse, dans laquelle l'orifice de sortie d'aérosol (17) présente un diamètre d'orifice de sortie d'aérosol (21), un axe central (22), et présente un bord circonférentiel (19) à partir duquel un canal de sortie d'aérosol (49) de la buse s'étend à partir de la surface de buse extérieure (18) vers un espace d'aérosolisation (20) à l'intérieur de la buse (1) en reliant l'espace d'aérosolisation (20) à la surface de buse extérieure (18) ;
un canal d'alimentation en fluide (14) avec un orifice de sortie de fluide (6) qui est aligné sur le même axe central (22) que l'orifice de sortie d'aérosol (17) et s'ouvre dans l'espace d'aérosolisation (20) à l'intérieur de la buse à une distance (24) d'une entrée (53) de l'espace d'aérosolisation (20) au canal de sortie d'aérosol (49), le diamètre d'orifice de sortie d'aérosol (21) étant au moins 1,3 fois plus grand qu'un diamètre d'orifice de sortie de fluide (23) ;
un canal de gaz sous pression annulaire convergent (27) s'étendant selon un angle d'écoulement de gaz aigu (61) par rapport à l'axe central (22), ledit canal de gaz sous pression annulaire convergent (27) présentant une sortie de gaz sous pression (51) au niveau de l'orifice de sortie de fluide (6) de sorte que du gaz sous pression sortant par la sortie de gaz sous pression (51) croise le fluide sortant de l'orifice de sortie de fluide (6) ; dans lequel le canal de gaz sous pression annulaire convergent (27) s'étend entre un insert de buse d'alimentation en fluide en forme de cône (4) de la buse, ledit insert de buse d'alimentation en fluide en forme de cône (4) présentant une paroi conique (56), et un insert avant (3) de la buse, ledit insert avant (3) présentant une paroi d'insert avant interne (28), dans lequel ledit canal de gaz sous pression annulaire convergent (27) comprend un premier canal annulaire partiel (55) et un second canal annulaire partiel (59), le premier canal annulaire partiel (55) présentant des parois interne et externe formées par la paroi conique (56) et la paroi d'insert avant interne (28) le long desquelles le gaz sous pression s'écoule avec l'angle d'écoulement de gaz aigu (61) défini par un angle de paroi conique (25) tel que mesuré entre la paroi conique (56) et l'axe central (22) et un angle de paroi d'insert avant (60) tel que mesuré entre la paroi d'insert avant interne (28) et l'axe central (22) au niveau de l'orifice de sortie de fluide (6), dans lequel l'angle d'écoulement de gaz aigu (61) est la somme de l'angle de paroi conique (25) et de l'angle de paroi d'insert avant (60) divisée par deux ;
le canal d'alimentation en fluide (14) est prévu à l'intérieur de l'insert de buse d'alimentation en fluide en forme de cône (4) formant la paroi conique (56) qui est la paroi interne du premier canal annulaire partiel (55), conçu sous la forme d'un canal de gaz comprimé circonférentiel convergent qui est centré axialement dans un espace creux en forme de cône (50) de l'insert avant (3) formant la paroi d'insert avant interne (28) qui est la paroi externe du premier canal annulaire partiel (55) ; **caractérisé en ce que**
l'angle d'écoulement de gaz aigu (61) est compris entre 10 et 30° ; et
la distance (24) entre l'orifice de sortie de fluide (6) et l'entrée (53) du canal de sortie d'aérosol (49) est supérieure à deux fois le diamètre d'orifice de sortie de fluide (23) et dans une plage comprise entre 100 µm et 2 mm.

2. Buse selon la revendication 1, dans laquelle une longueur de canal de sortie d'aérosol (30) du canal de sortie d'aérosol (49) est inférieure au diamètre d'orifice de sortie d'aérosol (21), et dans laquelle le diamètre d'orifice de sortie d'aérosol (21) est au moins 1,5 fois plus grand que le diamètre d'orifice de sortie de fluide (23).

3. Buse selon l'une quelconque des revendications précédentes, dans laquelle une zone de surface de buse extérieure (18) est convexe au niveau de l'orifice de sortie d'aérosol (17).

4. Buse selon l'une quelconque des revendications précédentes, dans laquelle l'espace d'aérosolisation (20) présente la forme d'un cône tronqué présentant son petit diamètre au niveau de l'entrée (53) vers le canal de sortie d'aérosol (49).

5. Buse selon l'une quelconque des revendications précédentes, dans laquelle au moins l'une de la paroi d'insert avant interne (28), d'une paroi d'insert de buse d'alimentation en fluide en forme de cône externe (29) de l'insert de buse d'alimentation en fluide en forme de cône (4), d'un canal de sortie d'aérosol (49) et d'une zone de surface de buse externe (18) est hydrophobe, omniphobe ou lyophobe.

6. Buse selon l'une quelconque des revendications précédentes, dans laquelle le premier canal annulaire partiel (55) diverge selon un angle de divergence aigu (58) compris entre 1 et 30°, l'angle de divergence aigu (58) étant la différence entre l'angle de paroi conique (25) et l'angle de paroi d'insert avant (60).

7. Buse selon la revendication 1, dans laquelle la buse est configurée pour :
aérosoliser un fluide avec une viscosité supérieure à 4 cSt ; et
aérosoliser le fluide à un débit supérieur à 1 ml/minute, en générant ainsi un aérosol contenant un médicament à un débit massique d'au moins 30 mg/minute sous forme de particules de fluide présentant un diamètre aérodynamique moyen en masse (MMAD) de 6 µm ou moins.

8. Buse selon la revendication 7, configurée en outre pour former l'aérosol à l'intérieur de l'espace d'aérosolisation (20) et pour sortir de cet espace d'aérosolisation (20) via l'aérosol entouré à l'intérieur d'une gaine de gaz largement dépourvue de particules d'aérosol.

9. Buse selon les revendications 7 à 8, configurée en outre pour pulvériser un fluide présentant une viscosité comprise entre 4 et 40 cSt, dans laquelle le diamètre aérodynamique moyen en masse (MMAD) des particules générées dans cette plage de viscosité est jusqu'à 40 % supérieur lorsque l'aérosol est généré à partir d'un fluide présentant une viscosité de 40 cSt par rapport à un fluide présentant une viscosité de 4 cSt.

10. Buse selon l'une quelconque des revendications 7 à 9, configurée en outre pour aérosoliser un fluide qui est une suspension de tensioactif.

11. Buse selon l'une quelconque des revendications 7 à 10, configurée en outre pour générer des aérosols de particules à partir de solutions avec des viscosités supérieures à 40 cSt, ou comprenant la génération d'aérosols de particules fines à partir de suspensions aqueuses avec des tensions superficielles comprises entre 2 et 72 mN/m.

12. Buse selon l'une quelconque des revendications 7 à 11, configurée pour entraver ou empêcher le reflux du fluide d'aérosolisation et un dysfonctionnement potentiel de la buse en choisissant un angle de divergence aigu (58) entre 1 et 30° selon lequel le premier canal annulaire partiel (55) diverge, ledit angle de divergence aigu (58) étant la différence entre l'angle de paroi conique (25) et l'angle de paroi d'insert avant (60), et en rendant la paroi d'insert avant interne (28) et la paroi conique (56) du premier canal annulaire partiel (55) hydrophobe, omniphobe ou lyophobe.
